(19) Europäisches Patentamt
European Patent Office
Office européen des brevets

(11) EP 2 599 507 A1

(12) EUROPÄISCHE PATENTANMELDUNG

(43) Veröffentlichungstag:
05.06.2013 Patentblatt 2013/23

(51) Int Cl.:
A61L 9/14 (2006.01)   B05B 17/00 (2006.01)
B65D 83/00 (2006.01)   B05B 7/00 (2006.01)
B05B 7/08 (2006.01)

(21) Anmeldenummer: 11191787.8

(22) Anmeldetag: 02.12.2011

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME

(71) Anmelder: Primavera ProAir GmbH
87466 Oy-Mittelberg (DE)

(72) Erfinder: Drathschmidt, Axel
87648 Aitrang (DE)

(74) Vertreter: Manske, Jörg et al
FRITZ Patent- und Rechtsanwälte
Apothekerstrasse 55
59755 Arnsberg (DE)

(54) **Fluidzerstäuber und Beduftungsvorrichtung mit mindestens einem Fluidzerstäuber**

(57) Die Erfindung betrifft einen Fluidzerstäuber (1) zum Zerstäuben eines Fluids, insbesondere eines Duftstofffluids, umfassend

- eine Zerstäubungskammer (2) mit mindestens einer Fluidauslassöffnung (211), durch die zerstäubte Fluidtropfen in die Umgebung ausgetragen werden können, und

- zumindest eine Zweistoff-Düsenanordnung (8), die innerhalb der Zerstäubungskammer (2) untergebracht ist und ein über einen Gaseinlass (3) mit einer Druckgasquelle verbindbares erstes Röhrchen (80) mit einer Gasausströmöffnung (800), durch die ein von der Druckgasquelle zugeführtes, unter Druck stehendes gasförmiges Medium in die Zerstäubungskammer (2) einströmen kann, und ein über einen Fluideinlass (4) mit einem Fluidreservoir verbindbares zweites Röhrchen (81) mit einer Fluidaustragöffnung (810), durch die das während des Betriebs des Fluidzerstäubers (1) angesaugte Fluid in die Zerstäubungskammer (2) ausgetragen und durch Wechselwirkung mit dem gasförmigen Medium zerstäubt werden kann, wobei das erste Röhrchen (80) und das zweite Röhrchen (81) geneigt zueinander angeordnet sind,

wobei sich die Fluidaustragöffnung (810) über den Rand der Gasausströmöffnung (800) des ersten Röhrchens (80) hinweg erstreckt und eine Längsachse des ersten Röhrchens (80) mit einer Längsachse des zweiten Röhrchens (81) einen Winkel > 60° und < 90°, vorzugsweise einen Winkel > 65° und < 85°, insbesondere einen Winkel zwischen 70° und 80°, einschließt. Darüber hinaus betrifft die vorliegende Erfindung eine Beduftungsvorrichtung (10) mit mindestens einem Fluidzerstäuber (1).

Fig. 4

EP 2 599 507 A1

**Beschreibung**

Die vorliegende Erfindung bezieht sich auf einen Fluidzerstäuber zum Zerstäuben eines Fluids, insbesondere eines Duftstofffluids, umfassend eine Zerstäubungskammer mit mindestens einer Fluidauslassöffnung, durch die zerstäubte Fluidtropfen in die Umgebung ausgetragen werden können, und zumindest eine Zweistoff-Düsenanordnung, die innerhalb der Zerstäubungskammer untergebracht ist und ein über einen Gaseinlass mit einer Druckgasquelle verbindbares erstes Röhrchen mit einer Gasausströmöffnung, durch die ein von der Druckgasquelle zugeführtes, unter Druck stehendes gasförmiges Medium in die Zerstäubungskammer einströmen kann, und ein über einen Fluideinlass mit einem Fluidreservoir verbindbares zweites Röhrchen mit einer Fluidaustragöffnung, durch die das während des Betriebs des Fluidzerstäubers angesaugte Fluid in die Zerstäubungskammer ausgetragen und durch Wechselwirkung mit dem gasförmigen Medium zerstäubt werden kann, wobei das erste Röhrchen und das zweite Röhrchen geneigt zueinander angeordnet sind. Darüber hinaus betrifft die vorliegende Erfindung eine Beduftungsvorrichtung, die insbesondere für eine Großraumbeduftung geeignet ist, umfassend ein Gehäuse, innerhalb dessen mindestens ein Fluidzerstäuber zum Zerstäuben eines aus einem Duftstofffluidreservoir zugeführten Duftstofffluids, insbesondere eines Duftparfüms oder ätherischen Duftöls, angeordnet ist, und mindestens einen Fluidauslass, durch den zerstäubte Duftstofffluidtropfen in die Umgebung ausgetragen werden können.

Aus dem Stand der Technik sind vergleichsweise einfach aufgebaute Beduftungsvorrichtungen zur Beduftung von Räumen bekannt, die auf einem Verdunstungsprinzip basieren und entweder eine integrierte Wärmequelle zur Verdunstung eines in einem offenen Gefäß vorgehaltenen Duftstofffluids, insbesondere eines Duftparfüms, ätherischen Duftöls oder eines Duftgels, aufweisen oder an eine externe Wärmequelle anschließbar sind. Kompakte Beduftungsvorrichtungen dieser Art sind auf Grund ihrer eingeschränkten Verdunstungsfläche nur für vergleichsweise kleine Räume geeignet. Ferner ist das Vorhalten des Duftstofffluids in einem offenen Gefäß ungünstig. Die Duftstofffluide sind häufig temperaturempfindlich und benötigen teilweise zusätzliche Lösungsmittel für die Verdampfung, so dass die Duftqualität nachteilig beeinflusst werden kann.

Seit einigen Jahren sind darüber hinaus auch Beduftungsvorrichtungen bekannt, die nach dem so genannten Zerstäuberprinzip arbeiten und mindestens einen Fluidzerstäuber aufweisen. Ein Beispiel für eine derartige Beduftungsvorrichtung mit einem Fluidzerstäuber offenbart das deutsche Gebrauchsmuster DE 200 23 948 U1. Der Fluidzerstäuber weist eine Zerstäubungskammer auf, innerhalb derer eine Zweistoff-Düsenanordnung mit einer Zufuhrleitung für das Duftstofffluid und einer Zufuhrleitung für ein Zerstäubungshilfsmedium (insbesondere Druckluft), die unter einem rechten Winkel zueinander angeordnet sind, untergebracht ist. Die Druckluft streicht dabei über die Austrittsöffnung der Duftstofffluidzufuhrleitung, so dass der Duftstoff aus der Zufuhrleitung angesaugt und zerstäubt wird. Dieser Fluidzerstäuber besteht vorzugsweise vollständig aus Glas und liefert in der Praxis ein extrem polydisperses Tropfenspektrum mit relativ groben Tropfen. Diese sind maßgeblich dafür verantwortlich, dass ein Großteil des zerstäubten Fluids abgeschieden und in das Fluidreservoir zurückgeführt wird.

Der vorliegenden Erfindung liegt die Aufgabe zugrunde, einen Fluidzerstäuber sowie eine Beduftungsvorrichtung mit mindestens einem Fluidzerstäuber zur Verfügung zu stellen, die eine hohe Effizienz mit einem geringen Fluidverbrauch sowie einem hohen energetischen Wirkungsgrad haben.

Diese Aufgabe wird durch einen Fluidzerstäuber mit den Merkmalen des kennzeichnenden Teils des Anspruchs 1 gelöst. Hinsichtlich der Beduftungsvorrichtung wird diese Aufgabe durch eine Beduftungsvorrichtung mit den Merkmalen des kennzeichnenden Teils des Anspruchs 12 gelöst. Zweckmäßige Weiterbildungen der Erfindung sind in den abhängigen Unteransprüchen angegeben.

Ein erfindungsgemäßer Fluidzerstäuber, der zum Beispiel als Parfümzerstäuber oder in einer Beduftungsvorrichtung zur Raumbeduftung eingesetzt werden kann, zeichnet sich dadurch aus, dass sich die Fluidaustragöffnung über den Rand der Gasausströmöffnung des ersten Röhrchens hinweg erstreckt und eine Längsachse des ersten Röhrchens mit einer Längsachse des zweiten Röhrchens einen Winkel > 60° und < 90°, vorzugsweise einen Winkel > 65° und < 85°, insbesondere einen Winkel zwischen 70° und 80°, einschließt. Die Erfindung geht von der Überlegung aus, einen Fluidzerstäuber bereitzustellen, dessen sogenanntes Primärspray einen hohen Anteil an Fluidtropfen mit sehr geringen Durchmessern aufweist, die auf Grund ihrer geringen Größe nicht auf die Innenwand der Zerstäubungskammer treffen und letztlich in das Fluidreservoir zurückfließen, sondern tatsächlich in die umgebende Atmosphäre gelangen können und somit wirksam zu deren Beduftung beitragen können. Ein hoher Anteil des tatsächlich aus dem Fluidzerstäuber in Form feinster Tröpfchen austretenden Fluids trägt auch zu einer effizienten Ausnutzung des zum Zerstäuben genutzten, unter Druck stehenden gasförmigen Mediums und somit zu einem energieeffizienten Betrieb bei. Auf Grund dieser Überlegungen schließt die Erfindung weiterhin den Gedanken ein, von den bisher eingesetzten Glaszerstäubern konventioneller Art abzugehen. Der erfindungsgemäße Aufbau des Fluidzerstäubers erbringt nicht nur, wie experimentelle Untersuchungen zeigen, ein Primärspray mit einem wesentlich erhöhten Anteil an feinen Fluidtropfen, sondern ist auch konstruktiv einfach und trägt somit zu niedrigen Gestehungskosten bei. Außerdem ermöglicht es diese Konstruktion, auch bei niedrigen Gasdifferenzdrücken und relativ niedrigem Luftvolumenstrom ein hinreichend feines Spray auszubilden - wenn auch bei höher viskosen Fluiden (Duftstofffluiden) derartige Betriebsbedingungen üblicherweise nicht eingestellt werden.

In einer bevorzugten Ausführungsform wird vorgeschlagen, dass die Gasausströmöffnung des ersten Röhrchens und die Fluidaustragöffnung des zweiten Röhrchens derart zueinander positioniert sind, dass der zur Gasausströmöffnung nächstgelegene Punkt der Fluidaustragöffnung auf einer Projektion des Umfangs der Gasausströmöffnung liegt oder sich bis etwa zum halben Radius innerhalb der Gasausströmöffnung erstreckt. Es hat sich gezeigt, dass durch diese Maßnahme die Zerstäubung des Duftstofffluids besonders effizient erfolgen kann.

In einer konstruktiv besonders einfachen Ausführungsform kann vorgesehen sein, dass das erste Röhrchen und/oder das zweite Röhrchen als gerade Hohlzylinder mit sich geradlinig erstreckenden Längsachsen ausgebildet sind/ist. Hohlzylindrische Röhrchen können in hoher Fertigungsgüte sehr einfach und kostengünstig hergestellt werden. In einer alternativen vorteilhaften Ausführungsform besteht die Möglichkeit, dass das erste Röhrchen mindestens einen gebogenen Abschnitt sowie einen sich geradlinig erstreckenden Endabschnitt mit der Gasausströmöffnung umfasst und/oder dass das zweite Röhrchen mindestens einen gebogenen Abschnitt sowie einen sich geradlinig erstreckenden Endabschnitt mit der Fluidaustragöffnung umfasst. Die Längsachsen, auf die sich gemäß Anspruch 1 die Angabe des Winkels zwischen den Röhrchen bezieht, werden dabei durch die Längsachsen der sich geradlinig erstreckenden (vorzugsweise hohlzylindrischen) Endabschnitte definiert. Es ist überdies möglich, über ihre gesamte Erstreckung gebogene (auch S-förmig gebogene oder gewendelte) erste und/oder zweite Röhrchen einzusetzen, um speziellen Anwendungsbedingungen Rechnung zu tragen oder spezielle Geräteaufbauten zu ermöglichen. Derartige Konstruktionen sind aber grundsätzlich aufwändiger und somit kostenintensiver.

Um das Zerstäubungsergebnis zu verbessern, kann in einer bevorzugten Ausführungsform die Fluidaustragöffnung des zweiten Röhrchens etwa 0,1 bis 0,7 mm, insbesondere 0,2 bis 0,5 mm, vor der Gasausströmöffnung des ersten Röhrchens angeordnet sein.

In einer besonders bevorzugten Ausführungsform besteht die Möglichkeit, dass der Durchmesser des ersten Röhrchens zwischen 0,8 und 1,2 mm liegt, insbesondere etwa 1,0 mm beträgt, und/oder dass der Durchmesser des zweiten Röhrchens zwischen 0,8 und 1,2 mm liegt, insbesondere etwa 1,0 mm beträgt. Dadurch kann zum Beispiel wirksam verhindert werden, dass das zweite Röhrchen während des Betriebs des Fluidzerstäubers von dem Duftstofffluid verstopft wird. Es soll an dieser Stelle betont werden, dass auch in der Dimensionierung von den vorstehend genannten Werten abweichende Realisierungen möglich sind.

Es ist vorteilhaft, dass die Länge des ersten Röhrchens oder des sich geradlinig erstreckenden Endabschnitts des ersten Röhrchens im Bereich zwischen 3 mm und 7 mm liegt, insbesondere etwa 5 mm beträgt, und/oder dass die Länge des zweiten Röhrchens oder des sich geradlinig erstreckenden Endabschnitts des zweiten Röhrchens im Bereich zwischen 6 mm und 10 mm liegt, insbesondere etwa 8 mm beträgt. Auch hier sind Abweichungen in Abhängigkeit vom konkreten Anwendungsfall und einer speziellen Gerätekonstruktion möglich und als im Bereich der Erfindung liegend anzusehen.

Um die Anzahl der herzustellenden Einzelteile des Fluidzerstäubers zu verringern, sieht eine vorteilhafte Ausführungsform vor, dass das erste Röhrchen integral mit dem Gaseinlass ausgebildet ist und/oder dass das zweite Röhrchen integral mit dem Fluideinlass ausgebildet ist.

In einer weiteren vorteilhaften Ausführungsform besteht die Möglichkeit, dass der Gaseinlass einen Röhrchen-Aufnahmekörper umfasst, in den das erste Röhrchen mit einem der Gasausströmöffnung gegenüberliegenden Ende eingesetzt ist und/oder dass der Fluideinlass einen Röhrchen-Aufnahmekörper umfasst, in den das zweite Röhrchen mit einem der Fluidaustragöffnung gegenüberliegenden Ende eingesetzt ist.

In einer besonders vorteilhaften Ausführungsform wird vorgeschlagen, dass im Inneren der Zerstäubungskammer oberhalb der Zweistoff-Düsenanordnung ein Trichtermittel angeordnet ist, das zu seinem unteren Ende hin verjüngt ausgebildet ist und an diesem unteren Ende eine Öffnung aufweist. Das Trichtermittel kann zum Beispiel kegelstumpfartig mit einer kreisförmigen oder ovalen Öffnung ausgebildet sein. Das Trichtermittel kann beispielsweise auch pyramidenstumpfartig mit einer rechteckigen (insbesondere quadratischen) Öffnung ausgebildet sein. Versuche haben gezeigt, dass ein derartiges Trichtermittel das Zerstäubungsergebnis weiter verbessern kann. Das Trichtermittel kann zumindest teilweise verhindern, dass zerstäubte Fluidtropfen mit vergleichsweise großen Durchmessern in einen Raumbereich oberhalb des Trichtermittels gelangen können. Ferner ermöglicht es die sich nach unten hin verjüngende Ausbildung des Trichtermittels, dass zerstäubte Fluidtropfen, die oberhalb des Trichtermittels nicht aus der Zerstäubungskammer ausgetragen werden und an den Innenwänden der Zerstäubungskammer abgeschieden werden und an diesen herunterlaufen können, wieder dem Fluidreservoir zugeführt und wiederverwertet werden können. Die Öffnung des Trichtermittels fluchtet vorzugsweise mit einem Fluidrücklauf, der (vorzugsweise bei allen Ausführungsformen) in einem Boden der Zerstäubungskammer ausgebildet ist, so dass das Fluid von der Öffnung des Trichtermittels auf Grund der Wirkung der Schwerkraft in den Fluidrücklauf gelangen kann und dadurch dem Fluidreservoir zugeführt werden kann. Der Boden der Zerstäubungskammer ist vorzugsweise nicht eben, sondern in Richtung des Fluidrücklaufs angeschrägt ausgebildet, so dass auf den Boden gelangendes Fluid in den Fluidrücklauf fließen kann.

In einer besonders vorteilhaften Ausführungsform kann vorgesehen sein, dass in der Fluidauslassöffnung eine Anschlusseinrichtung mit einem ersten Anschlussmittel, das mit der Druckgasquelle verbindbar ist, und mit mindestens einem zweiten Anschlussmittel angeordnet ist. Durch den Anschluss des ersten Anschlussmittel an die Druckgasquelle kann der Austrag der zerstäubten Fluidtropfen aus der Zerstäubungskammer wirksam verbessert werden.

Der hier vorgestellte Fluidzerstäuber ist auf Grund der geometrischen Ausgestaltung und Anordnung der beiden Röhrchen der Zweistoff-Düsenanordnung dazu in der Lage, bei geringem Energieverbrauch ein hinreichend feines Primärspray zu erzeugen. Der Fluidzerstäuber ist derart optimiert ausgeführt, dass ein hinreichend hoher, jedoch nicht allzu großer Volumenstrom des zu zerstäubenden Fluids angesaugt wird und dieser möglichst fein zerstäubt wird. Versuche haben gezeigt, dass mit Hilfe des hier vorgestellten Fluidzerstäubers Tropfenspektren mit tendenziell sehr kleinen Tropfendurchmessern erzeugt werden können. Dies ist selbst bei einem vergleichsweise geringen Differenzdruck $\Delta$p von etwa 0,2 bar und einem Volumenstrom des gasförmigen Mediums von etwa 1100 l/h möglich. Bei Versuchen mit unterschiedlichen Duftstofffluiden waren die Mittelwerte der zerstäubten Duftstofffluidtropfen < 1 $\mu$m und die Modalwerte < 1,5 $\mu$m. Im Ergebnis können mittels des hier vorgestellten Fluidzerstäubers sehr feine Duftstofffluidtropfen erhalten werden. Im Interesse einer hohen Betriebssicherheit ist es vorteilhaft, wenn der Fluidzerstäuber mit einem Differenzdruck $\Delta$p von mindesten 0,25 bar und einem Volumenstrom des gasförmigen Mediums von mindestens etwa 1450 l/h betrieben wird.

Eine erfindungsgemäße Beduftungsvorrichtung zeichnet sich dadurch aus, dass der mindestens eine Fluidzerstäuber nach einem der Ansprüche 1 bis 11 ausgebildet ist. Die erfindungsgemäße Beduftungsvorrichtung weist eine hohe Effizienz mit einem geringen Duftstoffverbrauch sowie einem hohen energetischen Wirkungsgrad auf.

In einer besonders vorteilhaften Ausführungsform wird vorgeschlagen, dass die Beduftungsvorrichtung ein Kompressormittel umfasst, das eine Druckgasquelle zum Betrieb des mindestens einen Fluidzerstäubers bildet. Das Kompressormittel, das während des Betriebs Luft aus der Umgebung ansaugen und verdichten kann, ist über (mindestens) eine Druckgasleitung an den Gaseinlass des Fluidzerstäubers sowie vorzugsweise darüber hinaus auch an das erste Anschlussmittel der an der Oberseite des Fluidzerstäubers in der Fluidauslassöffnung angeordneten Anschlusseinrichtung angeschlossen. Vorzugsweise ist eine einzelne Druckgasleitung vorgesehen, die an das erste Anschlussmittel angeschlossen ist und eine Abzweigleitung zum Anschluss an den Gaseinlass des Fluidzerstäubers aufweist. Alternativ können eine erste Druckgasleitung zum Anschluss des Kompressormittels an das erste Anschlussmittel der Anschlusseinrichtung sowie eine separate zweite Druckgasleitung zum Anschluss des Kompressormittels an den Gaseinlass des Fluidzerstäubers vorgesehen sein. Optional besteht bei jeder dieser Ausführungsvarianten die Möglichkeit, dass dem Gaseinlass des Fluidzerstäubers leitungsseitig ein Ventilmittel vorgeschaltet ist, das wahlweise geöffnet oder geschlossen werden kann, um dadurch die Druckgaszufuhr zum Gaseinlass des Fluidzerstäubers wahlweise aktivieren beziehungsweise deaktivieren oder auch regulieren zu können. Dem ersten Anschlussmittel der Anschlusseinrichtung ist vorzugsweise leitungsseitig ein Ventilmittel vorgeschaltet, das wahlweise geöffnet oder geschlossen werden kann, um dadurch die Druckgaszufuhr zum ersten Anschlussmittel der in der Fluidauslassöffnung des Fluidzerstäubers angeordneten Anschlusseinrichtung wahlweise aktivieren beziehungsweise deaktivieren oder auch regulieren zu können.

In einer besonders vorteilhaften Ausführungsform wird vorgeschlagen, dass die Beduftungsvorrichtung mindestens eine Duftstofffluidflasche aufweist, die das Duftstofffluidreservoir bildet und vorzugsweise mindestens ein Schnellverschlussmittel zum Anschluss mindestens einer Fluidleitung, insbesondere einer Fluidzufuhrleitung und/oder einer Fluidrücklaufleitung, aufweist. Schnellverschlussmittel ermöglichen in vorteilhafter Weise einen raschen und unter Handhabungsaspekten unkomplizierten Austausch der Duftstofffluidflasche, wenn zum Beispiel das Duftstofffluid verbraucht ist oder die Art des zu zerstäubenden Duftstofffluids gewechselt werden soll.

In einer besonders vorteilhaften Ausführungsform kann vorgesehen sein, dass zwischen der Fluidauslassöffnung des Fluidzerstäubers und dem Fluidauslass eine, vorzugsweise als gewickeltes Schlauchpaket ausgebildete, Beruhigungszone angeordnet ist, in die das zerstäubte Duftstofffluid einströmen und sich vor dem Austrag aus dem Fluidauslass beruhigen kann.

Der Betrieb der Beduftungsvorrichtung wird vorzugsweise mittels einer Steuerungseinrichtung, die zum Beispiel im Inneren des Gehäuses der Beduftungsvorrichtung angeordnet sein kann, gesteuert. Die Steuerungseinrichtung ist dazu in der Lage, die Beduftungsvorrichtung (insbesondere durch Öffnen und Schließen des Ventilmittels/der Ventilmittel) derart zu steuern, dass das Duftstofffluid innerhalb bestimmter voreingestellter oder auch von einem Benutzer voreinstellbarer Zerstäubungszyklen zerstäubt und ausgetragen wird. Zwischen zwei Zerstäubungszyklen, die ein (voreingestelltes oder voreinstellbares) Zeitintervall des Zerstäubens des Duftstofffluids und ein (voreingestelltes oder voreinstellbares) Zeitintervall des Austragens der Duftstofffluidtropfen aus der Beduftungsvorrichtung umfassen, erfolgt keine Zerstäubung. Die Länge des Zeitintervalls zwischen zwei Zerstäubungszyklen (Pausenlänge) kann ebenfalls entweder voreingestellt sein oder von einem Benutzer voreingestellt werden.

Die Beduftungsvorrichtung kann in einer weiteren vorteilhaften Ausführungsform ein Anschlussmittel (insbesondere ein Anschlusssteckermittel) umfassen, das den Anschluss eines Strömungserfassungsmittels, welches Luftbewegungen in einer Rohrleitung einer Belüftungs- beziehungsweise Raumklimatisierungseinrichtung erfassen kann, an die Beduftungsvorrichtung ermöglicht. Die Steuerungseinrichtung ist vorzugsweise so ausgebildet, dass sie das Einblasen der zerstäubten Duftstofffluidtropfen in die Rohrleitung der Belüftungsbeziehungsweise Raumklimatisierungseinrichtung nur dann freigibt, wenn von dem Strömungserfassungsmittel eine Luftbewegung in der Rohrleitung erkannt wird. Dadurch kann in vorteilhafter Weise verhindert werden, dass sich Duftstoff bei einer Störung oder bei ausgeschalteter Belüftungs- beziehungsweise Raumklimatisierungseinrichtung in der Rohrleitung anreichern kann und dann bei einem erneuten Einschalten der Belüftungsbeziehungsweise Raumklimatisierungseinrichtung eine zu intensive Beduftung erfolgt. Wenn die Beduftungsvorrichtung nicht an eine Belüftungs- beziehungsweise Raumklimatisierungseinrichtung angeschlossen wird, wird das Anschlussmittel geeignet gebrückt.

Weitere Merkmale und Vorteile der vorliegenden Erfindung werden deutlich anhand der nachfolgenden Beschreibung bevorzugter Ausführungsbeispiele unter Bezugnahme auf die beiliegenden Abbildungen. Dabei zeigen:

Fig. 1     eine perspektivische Ansicht eines Fluidzerstäubers, der gemäß einem bevorzugten Ausführungsbeispiel der vorliegenden Erfindung ausgeführt ist,

Fig. 2     eine Seitenansicht des Fluidzerstäubers gemäß Fig. 1 ,

Fig. 3     einen Schnitt durch den Fluidzerstäuber entlang einer Linie A-A in Fig. 2,

Fig. 4     eine vergrößerte Schnittdarstellung einer Zweistoff-Düsenanordnung des Fluidzerstäubers,

Fig. 5     eine Seitenansicht einer Beduftungsvorrichtung gemäß einem bevorzugten Ausführungsbeispiel der vorliegenden Erfindung mit einem Fluidzerstäuber gemäß Fig. 1 bis 4,

Fig. 6     eine weitere, in Bezug auf Fig. 5 um 90° gedrehte Seitenansicht der Beduftungsvorrichtung.

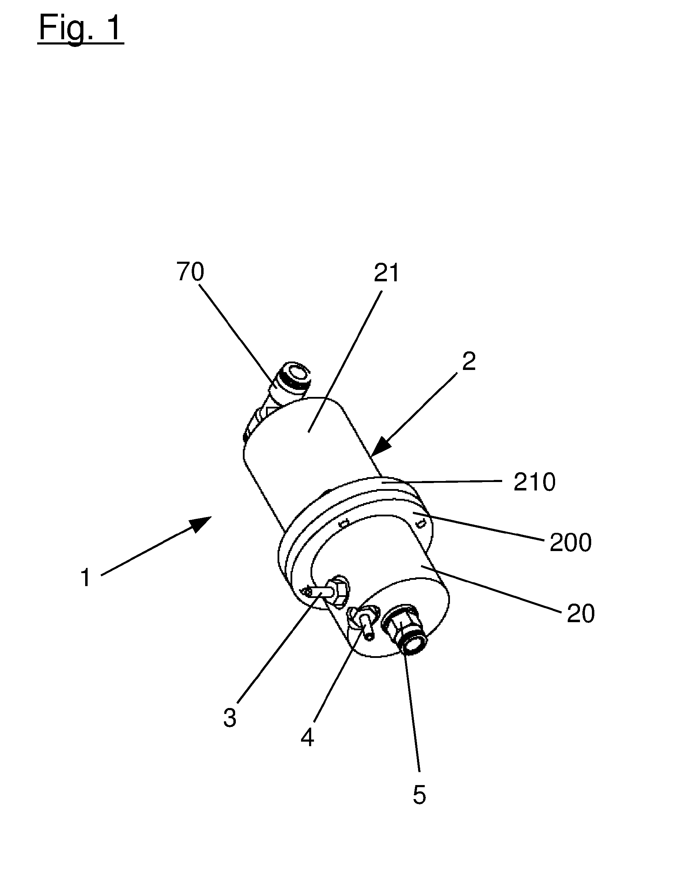

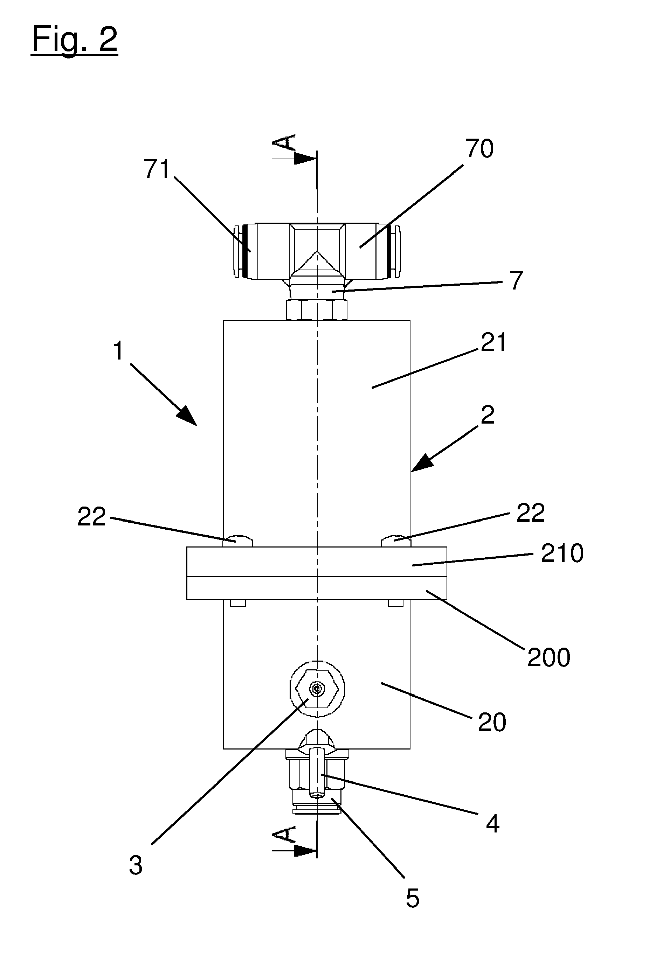

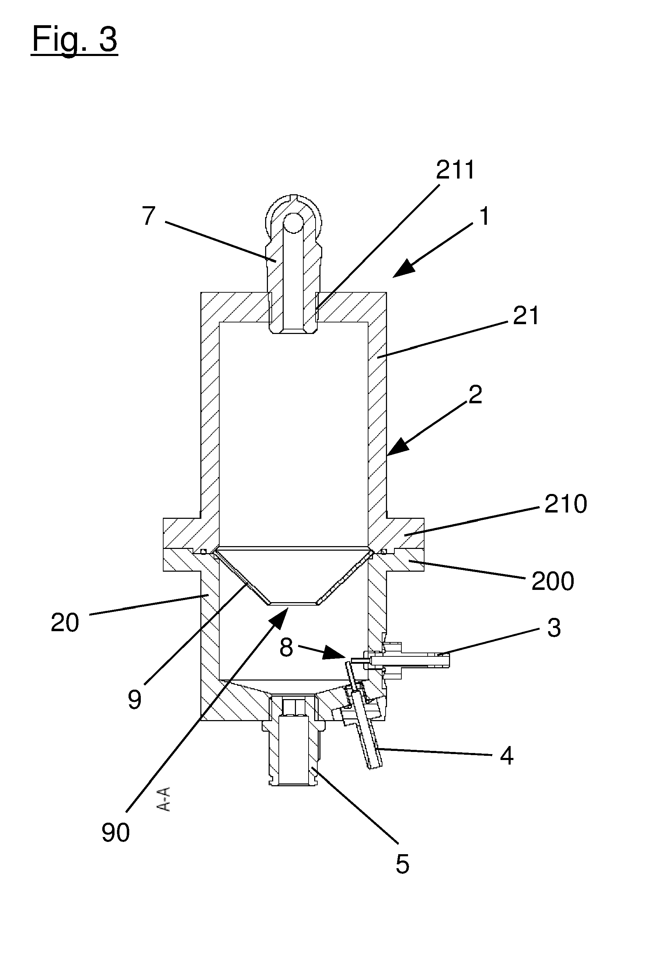

Unter Bezugnahme auf Fig. 1 bis 4 soll nachfolgend der grundlegende konstruktive Aufbau eines Fluidzerstäubers 1 , der gemäß einem bevorzugten Ausführungsbeispiel der vorliegenden Erfindung ausgeführt ist, näher erläutert werden.

Der Fluidzerstäuber 1 weist eine Zerstäubungskammer 2 auf, innerhalb derer ein Fluid, insbesondere ein Duftstofffluid (zum Beispiel ein Duftparfüm oder ätherisches Duftöl), zerstäubt werden kann. Die Zerstäubungskammer 2 ist in diesem Ausführungsbeispiel zweiteilig ausgebildet ist und weist ein erstes Zerstäubungskammerteil 20 und ein zweites Zerstäubungskammerteil 21 auf. Jedes der beiden Zerstäubungskammerteile 20, 21 weist in Umfangsrichtung einen ringförmigen Verbindungsflansch 200, 210 auf. Um die beiden Zerstäubungskammerteile 20, 21 miteinander zu verbinden, sind in jedem der beiden Verbindungsflansche 200, 210 in Umfangsrichtung verteilt mehrere Bohrungen ausgebildet. Bei der Montage werden die beiden Zerstäubungskammerteile 200, 210 aufeinandergesetzt und so ausgerichtet, dass jeweils eine Bohrung des Verbindungsflanschs 200 des ersten Zerstäubungskammerteils 20 mit einer damit korrespondierenden Bohrung des Verbindungsflanschs 210 des zweiten Zerstäubungskammerteils 21 fluchtet, so dass durch jedes der Bohrungspaare ein Befestigungsmittel 22 hindurchgeführt werden kann. Vorzugsweise bestehen die beiden Zerstäubungskammerteile 20, 21 aus Aluminium oder aus Edelstahl. Edelstahl beziehungsweise Aluminium haben den Vorteil, dass diese Werkstoffe nicht beziehungsweise nicht nennenswert mit dem zu zerstäubenden Duftstofffluid reagieren. Grundsätzlich können die beiden Zerstäubungskammerteile 20, 21 zum Beispiel auch aus einem Kunststoffmaterial hergestellt sein, welches vorzugsweise chemisch nicht beziehungsweise nicht nennenswert mit dem Duftstofffluid reagiert.

Das erste Zerstäubungskammerteil 20 weist in seiner Außenwand einen in diesem Ausführungsbeispiel stutzenartig ausgeführten Gaseinlass 3 auf, der mit einer Druckgasquelle verbindbar ist, so dass während des Betriebs des Fluidzerstäubers 1 über den Gaseinlass 3 ein unter Druck stehendes, gasförmiges Medium (insbesondere Druckluft) als Zerstäubungshilfsmedium in die Zerstäubungskammer 2 einströmen kann. Bodenseitig weist das erste Zerstäubungskammerteil 20 einen, vorliegend ebenfalls stutzenartigen Fluideinlass 4 auf, der mit einem Duftstofffluidreservoir verbindbar ist, so dass das zu zerstäubende Duftstofffluid, das zum Beispiel in Form eines ätherischen Duftöls vorliegen kann, durch den Fluideinlass 4 in die Zerstäubungskammer 2 einströmen kann. Ferner ist bodenseitig ein Fluidrücklauf 5 ausgebildet, der an das Duftstofffluidreservoir anschließbar ist, so dass ein Teil des zerstäubten Duftstofffluids, der nicht aus dem Fluidzerstäuber 1 in die Umgebung heraustritt, sondern insbesondere an einer Innenwand der Zerstäubungskammer 2 abgeschieden wird, durch den Fluidrücklauf 5 und eine entsprechende Fluidrücklaufleitung wieder in das Duftstofffluidreservoir zurückfließen kann und auf diese Weise wiederverwertet werden kann. Der Fluidrücklauf 5 ist in dem hier gezeigten Ausführungsbeispiel ebenfalls stutzenartig ausgebildet.

Wie weiter unten noch näher erläutert werden wird, wird das über den Fluideinlass 4 aus dem Duftstofffluidreservoir zugeführte Duftstofffluid mit Hilfe des unter Druck stehenden Gases, welches über den Gaseinlass 3 mit konstantem oder in einer alternativen Ausführungsform mit veränderbarem Druck zugeführt wird, zerstäubt und breitet sich sodann in der Zerstäubungskammer 2 aus und kann zumindest zum Teil aus einer Fluidauslassöffnung 211, die an einer Oberseite des zweiten Zerstäubungskammerteils 21 ausgebildet ist, in die Umgebung ausströmen. In der Fluidauslassöffnung 211 ist in dem hier vorgestellten Ausführungsbeispiel eine Anschlusseinrichtung 7 mit zwei Anschlussmitteln 70, 71 angeordnet. Die Anschlussmittel 70, 71 sind so ausgebildet, dass sie jeweils mit einer starr oder flexibel ausgebildeten Gas- beziehungsweise Fluidleitung verbunden werden können. Die Funktion der Anschlusseinrichtung 7 wird weiter unten noch näher erläutert.

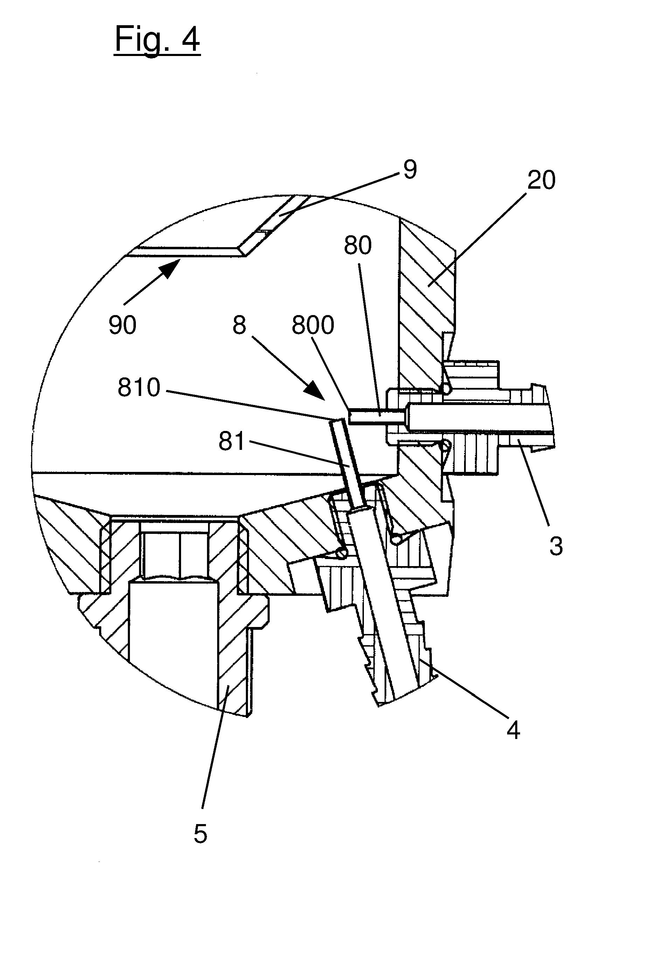

Zum Zerstäuben des Duftstofffluids weist der Fluidzerstäuber 1 eine Zweistoff-Düsenanordnung 8 auf, deren Konfiguration nachfolgend unter Bezugnahme auf Fig. 4 näher erläutert werden soll. Die Düsenanordnung 8 wird in diesem Ausführungsbeispiel durch ein erstes Röhrchen 80, das mit dem Gaseinlass 3 in Strömungsverbindung steht oder alternativ integral mit dem Gaseinlass 3 ausgeführt sein kann, und durch ein zweites Röhrchen 81, das mit dem Fluideinlass 4 in Strömungsverbindung steht oder alternativ integral mit dem Fluideinlass 4 ausgeführt sein kann, gebildet. Vorliegend mündet der Gaseinlass 3 in das erste Röhrchen 80 und der Fluideinlass 4 mündet in das zweite Röhrchen 81. In Strömungsrichtung des gasförmigen Mediums beziehungsweise Duftstofffluids ist beim Übergang vom Gaseinlass 3 in das erste Röhrchen 80 sowie vom Fluideinlass 4 in das zweite Röhrchen 81 jeweils ein Querschnittssprung hin zu einem kleineren Durchmesser zu erkennen. Auf diese Weise werden Düseneffekte erhalten.

Um eine Austauschbarkeit der Röhrchen 80, 81 zu ermöglichen, können der Gaseinlass 3 und der Fluideinlass 4 jeweils einen Röhrchenaufnahmekörper umfassen. In den Röhrchenaufnahmekörpern ist jeweils eines der beiden Röhrchen 80, 81 lösbar eingesetzt. Es besteht alternativ auch die Möglichkeit, dass die beiden Röhrchen 80, 81 unlösbar in den ihnen zugeordneten Röhrchenaufnahmekörpern angeordnet sind.

Das erste Röhrchen 80 ist über den Gaseinlass 3 mit der Druckgasquelle verbindbar und weist an seinem freien Ende eine Gasausströmöffnung 800 auf, durch die das zugeführte gasförmige Medium in das Innere der Zerstäubungskammer 2 strömen kann. Das zweite Röhrchens 81 ist über den Fluideinlass 4 mit dem Duftstofffluidreservoir verbindbar und weist an seinem freien Ende eine Fluidaustragöffnung 810 auf, durch die das zu zerstäubende Duftstofffluid während des Betriebs des Fluidzerstäubers 1 in das Innere der Zerstäubungskammer 2 ausgetragen und dabei durch Wechselwirkung mit dem gasförmigen Medium zerstäubt werden kann. Die beiden Röhrchen 80, 81 sind in dem hier gezeigten Ausführungsbeispiel als gerade Hohlzylinder ausgebildet und weisen somit eine Rotationssymmetrie um ihre jeweilige Längsachse auf. Vorliegend sind die beiden Röhrchen 80, 81 derart zueinander geneigt angeordnet, dass die Längsachsen der beiden Röhrchen 80, 81 einen Winkel von 75° miteinander einschließen. Der Winkel, unter dem sich die Längsachsen der beiden Röhrchen 80, 81 schneiden, ist in allen vorteilhaften Ausführungsformen > 60° und < 90° (vorzugsweise > 65° und < 85°, insbesondere zwischen 70° und 80°).

Wie in Fig. 4 zu erkennen, ragt die Fluidaustragöffnung 810 des zweiten Röhrchens 81 etwas über den Rand der Gasausströmöffnung 800 des ersten Röhrchens 80 hinweg. Während des Betriebs des Fluidzerstäubers 1 tritt aus der Gasausströmöffnung 800 des ersten Röhrchens 80 in Abhängigkeit von der freien Strömungsfläche A und dem herrschenden Differenzdruck $\Delta p$ ein Massenstrom $\dot{m}_G$ des über den Gaseinlass 3 zugeführten, unter Druck stehenden gasförmigen Mediums aus. Auf Grund der hohen Strömungsgeschwindigkeit $v_G$ des gasförmigen Mediums wird in einem Nahbereich der Gasausströmöffnung 800 ein definierter Saugdruck $p_s$ erzeugt, der dafür sorgt, dass das Duftstofffluid mit einem bestimmten Massenstrom $\dot{m}$ aus dem Duftstofffluidreservoir über die Fluidzufuhrleitung und den Fluideinlass 4 in das zweite Röhrchen 81 gefördert wird und beim Austritt aus der Fluidaustragöffnung 810 zerstäubt wird.

Der Massenstrom $\dot{m}$ hängt dabei physikalisch insbesondere von den Strömungsbedingungen in der Fluidzufuhrleitung, im Fluideinlass 4 sowie im zweiten Röhrchen 81 und somit insbesondere von der Reynolds-Zahl sowie der geodätischen Höhe ab. Daraus folgt, dass ein geringer angesaugter Massenstrom des Duftstofffluids bei konstanter Druckdifferenz $\Delta p$ und konstantem Strömungsquerschnitt eine geringe Beladung

$$\mu = \frac{\dot{m}_G}{\dot{m}}$$

zur Folge hat.

Während des Zerstäubens innerhalb des Fluidzerstäubers 1 werden Duftstofffluidtropfen in unterschiedlichen Größen erzeugt. Dabei nimmt die Feinheit des Tropfengrößenspektrums tendenziell mit zunehmender Beladung $\mu$ ab. Das einströmende gasförmige Medium wirkt in erster Linie mit einem sich am Rand der Fluidaustragöffnung 810 des zweiten Röhrchens 81 bildenden Fluidfilm zusammen und zerteilt diesen in polydisperse Tropfen, die sich in der Zerstäubungskammer 2 ausbreiten.

Für eine besonders wirksame Zerstäubung ist die Fluidaustragöffnung 810 des zweiten Röhrchens 81 vorzugsweise etwa 0,1 bis 0,7 mm, insbesondere 0,2 bis 0,5 mm, vor der Gasausströmöffnung 800 des ersten Röhrchens 80 angeordnet. Für ein gutes Zerstäubungsergebnis des Duftstofffluids hat es sich darüber hinaus als vorteilhaft erwiesen, wenn das erste Röhrchen 80 und das zweite Röhrchen 81 einen Durchmesser zwischen 0,8 und 1,2 mm, insbesondere einen Durchmesser von etwa 1,0 mm aufweisen. Eine Verstopfung des zweiten Röhrchens 81 mit dem Duftstofffluid kann dadurch in vorteilhafter Weise verhindert werden. Es soll an dieser Stelle betont werden, dass auch in der Dimensionierung von den vorstehend genannten Werten abweichende Realisierungen der Durchmesser und/oder Abstände sowie andere geometrische Formen des ersten und/oder zweiten Röhrchens 80, 81 möglich sind.

Wie in Fig. 4 zu erkennen, ist innerhalb der Zerstäubungskammer 2 (und zwar oberhalb der Düsenanordnung 8) ferner ein vorliegend kegelstumpfartig ausgebildetes Trichtermittel 9 angeordnet, das zu seinem unteren Ende hin verjüngt ausgebildet ist und an diesem unteren Ende eine zentrale, kreisförmige Öffnung 90 aufweist. Es hat sich gezeigt, dass ein derartiges Trichtermittel 9 das Zerstäubungsergebnis weiter verbessern kann. Das Trichtermittel 9 kann zumindest teilweise verhindern, dass zerstäubte Duftstofffluidtropfen mit vergleichsweise großen Durchmessern in einen Raumbereich oberhalb des Trichtermittels 9 gelangen können. Ferner ermöglicht es die konische Ausbildung des Trichtermittels 9, dass zerstäubte Duftstofffluidtropfen, die oberhalb des Trichtermittels 9 nicht aus der Zerstäubungskammer 2 ausgetragen werden und an den Innenwänden der Zerstäubungskammer 2 abgeschieden werden und an diesen herunterlaufen können, wieder dem Duftstofffluidreservoir zugeführt und wiederverwertet werden können. Die zentrale Öffnung 90 des Trichtermittels 9 fluchtet mit dem Fluidrücklauf 5, so dass das Duftstofffluid von der Öffnung 90 des Trichtermittels 9 auf Grund der Wirkung der Schwerkraft in den Fluidrücklauf 5 gelangen kann und dadurch dem Duftstofffluidreservoir zugeführt werden kann. Der Boden des ersten Zerstäubungskammerteils 20 ist seinerseits nicht eben, sondern in Richtung des Fluidrücklaufs 5 angeschrägt ausgebildet, so dass auf den Boden des ersten Zerstäubungskammerteils 20 gelangendes Duftstofffluid in den Fluidrücklauf 5 fließen kann.

Der hier vorgestellte Lösungsansatz geht von der Überlegung aus, einen Fluidzerstäuber 1 zur Verfügung zu stellen, bei dem das durch Zerstäuben des Duftstofffluids generierte, aus Duftstofffluidtropfen bestehende Primärspray einen hohen Anteil an Tropfen mit sehr geringen Durchmessern aufweist, die auf Grund ihrer geringen Größe tendenziell nicht auf die Innenwand der Zerstäubungskammer 2 treffen und letztlich in das Duftstofffluidreservoir zurückfließen, sondern tatsächlich in die umgebende Atmosphäre gelangen können und somit wirksam zu deren Beduftung beitragen können. Ein hoher Anteil des tatsächlich aus dem Fluidzerstäuber 1 in Form feinster Tropfen austretenden Duftstofffluids trägt auch zu einer effizienten Ausnutzung des zum Zerstäuben genutzten gasförmigen Mediums und somit zu einem energieeffizienten Betrieb einer entsprechenden Beduftungsvorrichtung 10, die nachfolgend noch näher erläutert werden wird, bei.

Der hier vorgestellte Fluidzerstäuber 1 ist auf Grund der geometrischen Ausgestaltung und Anordnung der beiden Röhrchen 80, 81 der Zweistoff-Düsenanordnung 8 dazu in der Lage, bei einer möglichst geringen Beladung (Kompressorleistung) ein hinreichend feines Primärspray zu erzeugen. Wie oben bereits erläutert, saugt die Zweistoff-Düsenanordnung 8 das zu zerstäubende Duftstofffluid aus dem Duftstofffluidreservoir an. Der Fluidzerstäuber 1 ist nunmehr derart optimiert ausgeführt, dass ein hinreichend hoher, jedoch nicht allzu großer Volumenstrom des zu zerstäubenden Duftstofffluids angesaugt wird und dieser möglicht fein zerstäubt wird. Versuche haben gezeigt, dass mit Hilfe des hier vorgestellten Fluidzerstäubers 1 Tropfenspektren mit tendenziell sehr kleinen Tropfendurchmessern erzeugt werden können. Dies ist selbst bei einem vergleichsweise geringen Differenzdruck ∆p von etwa 0,2 bar und einem Volumenstrom des gasförmigen Mediums von etwa 1100 l/h möglich. Bei Versuchen mit unterschiedlichen Duftstofffluiden waren die Mittelwerte der zerstäubten Duftstofffluidtropfen < 1 µm und die Modalwerte < 1,5 µm. Im Ergebnis können mittels des hier vorgestellten Fluidzerstäubers 1 sehr feine Duftstofffluidtropfen erhalten werden. Im Interesse einer hohen Betriebssicherheit ist es vorteilhaft, wenn der Fluidzerstäuber 1 mit einem Differenzdruck ∆p von mindesten 0,25 bar und einem Volumenstrom des gasförmigen Mediums von mindestens etwa 1450 l/h betrieben wird.

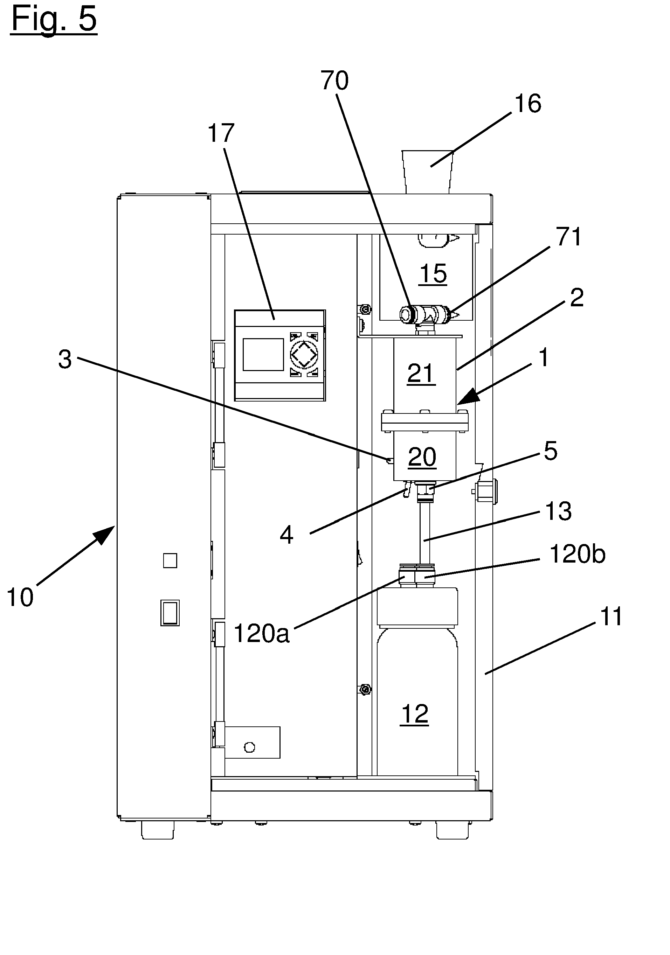

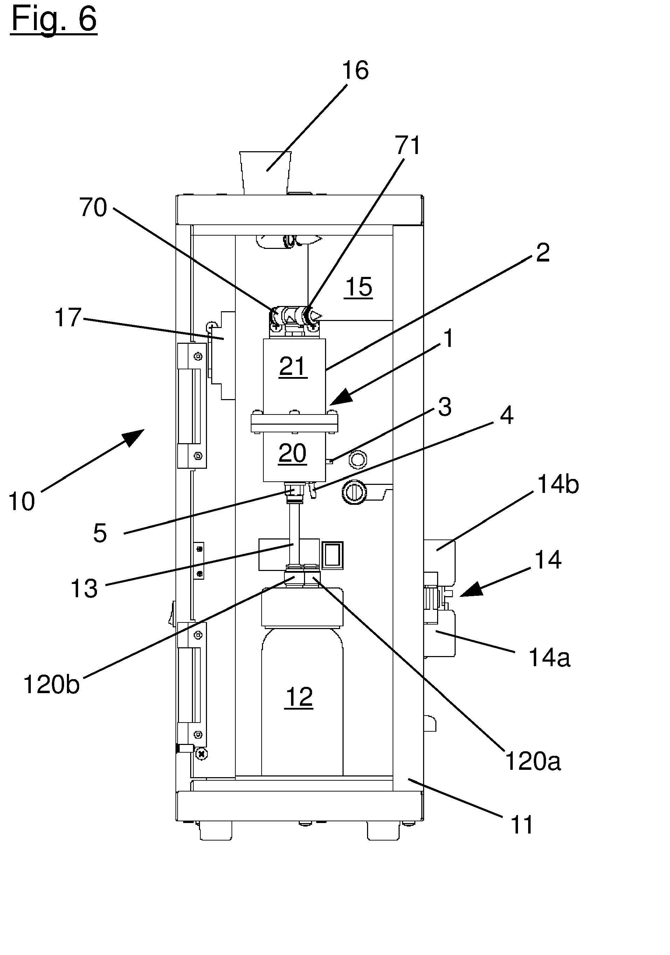

Unter Bezugnahme auf Fig. 5 und 6 weist eine Beduftungsvorrichtung 10, die gemäß einem bevorzugten Ausführungsbeispiel der vorliegenden Erfindung ausgeführt ist, ein Gehäuse 11 auf, innerhalb dessen der in Fig. 1 bis 4 gezeigte Fluidzerstäuber 1 angeordnet ist. Unterhalb des Fluidzerstäubers 1 ist eine Duftstofffluidflasche 12 angeordnet, die ein Duftstofffluidrervoir bildet und an ihrer Oberseite zwei Schnellverschlussmittel 120a, 120b aufweist. Ein erstes Schnellverschlussmittel 120a ist dabei für den Anschluss einer (hier nicht explizit dargestellten) Fluidzufuhrleitung vorgesehen, die flexibel (insbesondere als Schlauchleitung) oder auch starr ausgebildet sein kann und an den Fluideinlass 4 des Fluidzerstäubers 1 angeschlossen wird. Ein zweites Schnellverschlussmittel 120b ist für den Anschluss einer Fluidrücklaufleitung 13 vorgesehen, die flexibel (insbesondere als Schlauchleitung) oder auch starr ausgebildet sein kann und an den Fluidrücklauf 5 des Fluidzerstäubers 1 angeschlossen ist. Die Schnellverschlussmittel 120a, 120b ermöglichen in vorteilhafter Weise einen raschen und unter Handhabungsaspekten unkomplizierten Austausch der Duftstofffluidflasche 12, wenn zum Beispiel das Duftstofffluid verbraucht ist oder die Art des zu zerstäubenden Duftstofffluids gewechselt werden soll.

Ferner umfasst die Beduftungsvorrichtung 10 ein hier nicht explizit dargestelltes, vorzugsweise innerhalb des Gehäuses 11 angeordnetes Kompressormittel, welches eine Druckgasquelle für den Betrieb der Beduftungsvorrichtung 10 mit dem Fluidzerstäuber 1 zur Verfügung stellt. Das Kompressormittel, das während des Betriebs Luft aus der Umgebung ansaugen und verdichten kann, ist über (mindestens) eine Druckgasleitung (hier nicht explizit gezeigt) an den Gaseinlass 3 des Fluidzerstäubers 1 sowie an das erste Anschlussmittel 70 der an der Oberseite des Fluidzerstäubers 1 in die Fluidauslassöffnung 211 eingesetzten Anschlusseinrichtung 7 angeschlossen. Vorzugsweise ist eine einzelne Druckgasleitung vorgesehen, die an das erste Anschlussmittel 70 angeschlossen ist und eine Abzweigleitung zum Anschluss an den Gaseinlass 3 des Fluidzerstäubers 1 aufweist. Alternativ können eine erste Druckgasleitung zum Anschluss des Kompressormittels an das erste Anschlussmittel 70 der Anschlusseinrichtung 7 sowie eine separate zweite Druckgasleitung zum Anschluss des Kompressormittels an den Gaseinlass 3 des Fluidzerstäubers 1 vorgesehen sein. Optional besteht bei jeder dieser Ausführungsvarianten die Möglichkeit, dass dem Gaseinlass 3 des Fluidzerstäubers 1 leitungsseitig ein hier nicht explizit gezeigtes Ventilmittel vorgeschaltet ist, das wahlweise geöffnet oder geschlossen werden kann, um dadurch die Druckgaszufuhr zum Gaseinlass 3 des Fluidzerstäubers 1 wahlweise aktivieren beziehungsweise deaktivieren oder auch regulieren zu können.

Dem ersten Anschlussmittel 70 der Anschlusseinrichtung 7 ist leitungsseitig ein hier nicht explizit gezeigtes Ventilmittel vorgeschaltet, das wahlweise geöffnet oder geschlossen werden kann, um dadurch die Druckgaszufuhr zum ersten Anschlussmittel 70 der in der Fluidauslassöffnung 211 des Fluidzerstäubers 1 angeordneten Anschlusseinrichtung 7 wahlweise aktivieren beziehungsweise deaktivieren oder auch regulieren zu können. Das zweite Anschlussmittel 71 des Anschlussmittels 7 ist an eine Beruhigungszone 15 oberhalb des Fluidzerstäubers 1 angeschlossen. In diese Beruhigungszone 15 können die aus der Zerstäubungskammer 2 in die Anschlusseinrichtung 7 austretenden, zerstäubten Duftstofffluidtropfen, die von dem durch das erste Anschlussmittel 70 in die Anschlusseinrichtung 7 einströmenden und unter Druck stehenden gasförmigen Medium beaufschlagt werden, einströmen. Beim Durchströmen der Beruhigungszone 15 können sich die Duftstofffluidtropfen beruhigen und anschließend aus einem Fluidauslass 16, der an die Beruhigungszone 15 angeschlossen ist und vorliegend stutzenartig ausgebildet ist, aus der Beduftungsvorrichtung 10 unmittelbar in die Umgebung ausströmen, um diese in der gewünschten Weise zu beduften.

Die zerstäubten Duftstofffluidtropfen können zum Beispiel auch in eine an den Fluidauslass 16 anschließbare Belüftungsbeziehungsweise Raumklimatisierungseinrichtung einströmen, mittels derer sie in der gewünschten Weise in einem oder mehreren Räumen oder in bestimmten Zonen eines oder mehrerer Räume eines Gebäudes in der gewünschten Weise ausgetragen und verteilt werden können. In diesem Ausführungsbeispiel wird die Beruhigungszone 15 durch ein gewickeltes, insbesondere spiralförmig gewickeltes, Schlauchpaket gebildet, durch das die Duftstofffluidtropfen zum Fluidauslass 16 strömen können. Andere konstruktive Umsetzungen der Beruhigungszone 15, mittels derer die Duftstofffluidtropfen beruhigt werden können, sind ebenfalls möglich.

Der Betrieb der Beduftungsvorrichtung 10 wird mittels einer Steuerungseinrichtung 17, die in diesem Ausführungsbeispiel ebenfalls im Inneren des Gehäuses 11 der Beduftungsvorrichtung 10 angeordnet ist, gesteuert. Die Steuerungseinrichtung 17 ist dazu in der Lage, die Beduftungsvorrichtung 10 (insbesondere durch Öffnen und Schließen des Ventilmittels/der Ventilmittel) derart zu steuern, dass das Duftstofffluid innerhalb bestimmter voreingestellter oder auch von einem Benutzer voreinstellbarer Zerstäubungszyklen zerstäubt und ausgetragen wird. Zwischen zwei Zerstäubungszyklen, die ein (voreingestelltes oder voreinstellbares) Zeitintervall des Zerstäubens des Duftstofffluids und ein (voreingestelltes oder voreinstellbares) Zeitintervall des Austragens der Duftstofffluidtropfen aus der Beduftungsvorrichtung 10 umfassen, erfolgt keine Zerstäubung. Die Länge dieses Zeitintervalls zwischen zwei Zerstäubungszyklen (Pausenlänge) kann ebenfalls entweder voreingestellt sein oder von einem Benutzer voreingestellt werden. Die Beduftungsvorrichtung 10 weist in diesem Ausführungsbeispiel ferner ein vorliegend außerhalb des Gehäuses 11 angeordnetes Anschlusssteckermittel 14 auf, das den Anschluss eines Strömungserfassungsmittels, welches Luftbewegungen in einer Rohrleitung einer Belüftungsbeziehungsweise Raumklimatisierungseinrichtung erfassen kann, an die Beduftungsvorrichtung 10 ermöglicht. Das Anschlusssteckermittel 14 weist in diesem Ausführungsbeispiel ein erstes Anschlusssteckerteil 14a und ein zweites Anschlusssteckerteil 14b auf. Die Steuerungseinrichtung 17 ist vorzugsweise so ausgebildet, dass sie das Einblasen der zerstäubten Duftstofffluidtropfen in die Rohrleitung der Belüftungs- beziehungsweise Raumklimatisierungseinrichtung nur dann freigibt, wenn von dem Strömungserfassungsmittel eine Luftbewegung in der Rohrleitung erkannt wird. Dadurch kann in vorteilhafter Weise verhindert werden, dass sich Duftstoff bei einer Störung oder bei ausgeschalteter Belüftungs- beziehungsweise Raumklimatisierungseinrichtung in der Rohrleitung anreichern kann und dann bei einem erneuten Einschalten der Belüftungsbeziehungsweise Raumklimatisierungseinrichtung eine zu intensive Beduftung erfolgt. Wenn die Beduftungsvorrichtung 10 nicht an eine Belüftungs- beziehungsweise Raumklimatisierungseinrichtung angeschlossen wird, wird das zweite Anschlusssteckerteil 14b gebrückt.

## Patentansprüche

1.  Fluidzerstäuber (1) zum Zerstäuben eines Fluids, insbesondere eines Duftstofffluids, umfassend

- eine Zerstäubungskammer (2) mit mindestens einer Fluidauslassöffnung (211), durch die zerstäubte Fluidtropfen in die Umgebung ausgetragen werden können, und

- zumindest eine Zweistoff-Düsenanordnung (8), die innerhalb der Zerstäubungskammer (2) untergebracht ist und ein über einen Gaseinlass (3) mit einer Druckgasquelle verbindbares erstes Röhrchen (80) mit einer Gasausströmöffnung (800), durch die ein von der Druckgasquelle zugeführtes, unter Druck stehendes gasförmiges Medium in die Zerstäubungskammer (2) einströmen kann, und ein über einen Fluideinlass (4) mit einem Fluidreservoir verbindbares zweites Röhrchen (81) mit einer Fluidaustragöffnung (810), durch die das während des Betriebs des Fluidzerstäubers (1) angesaugte Fluid in die Zerstäubungskammer (2) ausgetragen und durch Wechselwirkung mit dem gasförmigen Medium zerstäubt werden kann, wobei das erste Röhrchen (80) und das zweite Röhrchen (81) geneigt zueinander angeordnet sind, **dadurch gekennzeichnet, dass** sich die Fluidaustragöffnung (810) über den Rand der Gasausströmöffnung (800) des ersten Röhrchens (80) hinweg erstreckt und eine Längsachse des ersten Röhrchens (80) mit einer Längsachse des zweiten Röhrchens (81) einen Winkel > 60° und < 90°, vorzugsweise einen Winkel > 65° und < 85°, insbesondere einen Winkel zwischen 70° und 80°, einschließt.

2. Fluidzerstäuber (1) nach Anspruch 1 , **dadurch gekennzeichnet, dass** die Gasausströmöffnung (800) des ersten Röhrchens (80) und die Fluidaustragöffnung (810) des zweiten Röhrchens (81) derart zueinander positioniert sind, dass der zur Gasausströmöffnung (800) nächstgelegene Punkt der Fluidaustragöffnung (810) auf einer Projektion des Umfangs der Gasausströmöffnung (800) liegt oder sich bis etwa zum halben Radius innerhalb der Gasausströmöffnung (800) erstreckt.

3. Fluidzerstäuber (1) nach einem der Ansprüche 1 oder 2,
   **dadurch gekennzeichnet, dass** das erste Röhrchen (80) und/oder das zweite Röhrchen (81) als gerade Hohlzylinder mit sich geradlinig erstreckenden Längsachsen ausgebildet sind/ist.

4. Fluidzerstäuber (1) nach einem der Ansprüche 1 oder 2,
   **dadurch gekennzeichnet, dass** das erste Röhrchen (80) mindestens einen gebogenen Abschnitt sowie einen sich geradlinig erstreckenden Endabschnitt mit der Gasausströmöffnung (800) umfasst und/oder dass das zweite Röhrchen (81) mindestens einen gebogenen Abschnitt sowie einen sich geradlinig erstreckenden Endabschnitt mit der Fluidaustragöffnung (810) umfasst.

5. Fluidzerstäuber (1) nach einem der Ansprüche 1 bis 4, **dadurch gekennzeichnet, dass** die Fluidaustragöffnung (810) des zweiten Röhrchens (81) etwa 0,1 bis 0,7 mm, insbesondere 0,2 bis 0,5 mm, vor der Gasausströmöffnung (800) des ersten Röhrchens (80) angeordnet ist.

6. Fluidzerstäuber (1) nach einem der Ansprüche 1 bis 5, **dadurch gekennzeichnet, dass** der Durchmesser des ersten Röhrchens (80) zwischen 0,8 und 1,2 mm liegt, insbesondere etwa 1,0 mm beträgt, und/oder dass der Durchmesser des zweiten Röhrchens (81) zwischen 0,8 und 1,2 mm liegt, insbesondere etwa 1,0 mm beträgt.

7. Fluidzerstäuber (1) nach einem der Ansprüche 1 bis 6, **dadurch gekennzeichnet, dass** die Länge des ersten Röhrchens (80) oder des sich geradlinig erstreckenden Endabschnitts des ersten Röhrchens (80) im Bereich zwischen 3 mm und 7 mm liegt, insbesondere etwa 5 mm beträgt, und/oder dass die Länge des zweiten Röhrchens (81) oder des sich geradlinig erstreckenden Endabschnitts des zweiten Röhrchens (81) im Bereich zwischen 6 mm und 10 mm liegt, insbesondere etwa 8 mm beträgt.

8. Fluidzerstäuber (1) nach einem der Ansprüche 1 bis 7, **dadurch gekennzeichnet, dass** das erste Röhrchen (80) integral mit dem Gaseinlass (3) ausgebildet ist und/oder dass das zweite Röhrchen (81) integral mit dem Fluideinlass (4) ausgebildet ist.

9. Fluidzerstäuber (1) nach einem der Ansprüche 1 bis 8, **dadurch gekennzeichnet, dass** der Gaseinlass (3) einen Röhrchen-Aufnahmekörper umfasst, in den das erste Röhrchen (80) mit einem der Gasausströmöffnung (800) gegenüberliegenden Ende eingesetzt ist und/oder dass der Fluideinlass (4) einen Röhrchen-Aufnahmekörper umfasst, in den das zweite Röhrchen (81) mit einem der Fluidaustragöffnung (810) gegenüberliegenden Ende eingesetzt ist.

10. Fluidzerstäuber (1) nach einem der Ansprüche 1 bis 9, **dadurch gekennzeichnet, dass** im Inneren der Zerstäubungskammer (2) oberhalb der Zweistoff-Düsenanordnung (8) ein Trichtermittel (9) angeordnet ist, das zu seinem unteren Ende hin verjüngt ausgebildet ist und an diesem unteren Ende eine Öffnung (90) aufweist.

11. Fluidzerstäuber (1) nach einem der Ansprüche 1 bis 10, **dadurch gekennzeichnet, dass** in der Fluidauslassöffnung (211) eine Anschlusseinrichtung (7) mit einem ersten Anschlussmittel (70), das mit der Druckgasquelle verbindbar ist, und mit mindestens einem zweiten Anschlussmittel (71) angeordnet ist.

12. Beduftungsvorrichtung (10), umfassend

- ein Gehäuse (11), innerhalb dessen mindestens ein Fluidzerstäuber (1) zum Zerstäuben eines aus einem Duftstofffluidreservoir zugeführten Duftstofffluids, insbesondere eines Duftparfüms oder ätherischen Duftöls, angeordnet ist, und
- mindestens einen Fluidauslass (16), durch den zerstäubte Duftstofffluidtropfen in die Umgebung ausgetragen werden können, **dadurch gekennzeichnet, dass** der mindestens eine Fluidzerstäuber (1) nach einem der Ansprüche 1 bis 11 ausgebildet ist.

13. Beduftungsvorrichtung (10) nach Anspruch 12, **dadurch gekennzeichnet, dass** die Beduftungsvorrichtung (10) ein Kompressormittel umfasst, das eine Druckgasquelle zum Betrieb des mindestens einen Fluidzerstäubers (1) bildet.

14. Beduftungsvorrichtung (10) nach Anspruch 12 oder 13, **dadurch gekennzeichnet, dass** die Beduftungsvorrichtung (10) mindestens eine Duftstofffluidflasche (12) aufweist, die das Duftstofffluidreservoir bildet und vorzugsweise mindestens ein Schnellverschlussmittel (120a, 120b) zum Anschluss mindestens einer Fluidleitung, insbesondere einer Fluidzufuhrleitung und/oder einer Fluidrücklaufleitung (13), aufweist.

15. Beduftungsvorrichtung (10) nach einem der Ansprüche 12 bis 14, **dadurch gekennzeichnet, dass** zwischen der Fluidauslassöffnung (211) des Fluidzerstäubers (1) und dem Fluidauslass (16) eine, vorzugsweise als gewickeltes Schlauchpaket ausgebildete, Beruhigungszone (15) angeordnet ist, in die das zerstäubte Duftstofffluid einströmen und sich vor dem Austrag aus dem Fluidauslass (16) beruhigen kann.

Fig. 1

# Fig. 2

# Fig. 3

# Fig. 4

# Fig. 5

# Fig. 6

**EP 2 599 507 A1**

Europäisches
Patentamt

European
Patent Office

Office européen
des brevets

**EUROPÄISCHER RECHERCHENBERICHT**

Nummer der Anmeldung

EP 11 19 1787

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 02/48616 A2 (AEROME GMBH SCENT SYSTEM ENGIN [DE]; BARTELS FRANK [DE]) 20. Juni 2002 (2002-06-20) * Abbildungen 2,3,5 * * Seite 11, Zeile 19 - Zeile 20 * ----- | 1-3,8,9, 11 | INV. A61L9/14 B05B17/00 B65D83/00 B05B7/00 B05B7/08 |
| X | DE 37 09 667 A1 (BAUER THEOPHIL [DE]) 13. Oktober 1988 (1988-10-13) * Zusammenfassung; Abbildung 2 * * Anspruch 1 * ----- | 1-3,8,9, 11 | |
| X | WO 2007/073825 A2 (BAYER MATERIALSCIENCE AG [DE]; HENNECKE GMBH [DE]; KLEBA INGO [DE]; WI) 5. Juli 2007 (2007-07-05) * Zusammenfassung; Abbildung 7 * * Winkel; Absatz [0043] * ----- | 1-3,8,9, 11 | |
| X | WO 2006/122561 A1 (GRUNDFOS MANAGEMENT AS [DK]; BOE CHRISTIAN [DK]) 23. November 2006 (2006-11-23) * Absatz [0058]; Abbildung 3 * ----- | 1-3,8,9, 11 | RECHERCHIERTE SACHGEBIETE (IPC) |
| X | US 5 569 073 A (COOPER GUY F [US]) 29. Oktober 1996 (1996-10-29) * Abbildung 1 * ----- | 1-3,8,9, 11 | A61L B05B B65D |
| X | EP 0 546 739 A2 (MOBIL OIL CORP [US]) 16. Juni 1993 (1993-06-16) * Abbildung 9 * * Zusammenfassung * ----- | 1-3,8,9, 11 | |
| X | DE 12 66 697 B (MOTAN G M B H) 18. April 1968 (1968-04-18) * Abbildung 1 * ----- | 1-3,8,9, 11 | |

-/--

~~Der vorliegende Recherchenbericht wurde für alle Patentans~~prüche erstellt

| Recherchenort | Abschlußdatum der Recherche | Prüfer |
|---|---|---|
| München | 5. April 2012 | Fischer, Michael |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

17

Europäisches
Patentamt

European
Patent Office

Office européen
des brevets

**EUROPÄISCHER RECHERCHENBERICHT**

Nummer der Anmeldung

EP 11 19 1787

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 200 23 948 U1 (AROMATA INTERNAT GMBH [DE]) 13. Dezember 2007 (2007-12-13) * das ganze Dokument * ----- | 1-3,8,9, 11 | |
| X | DE 103 39 545 A1 (ZAPF CREATION AG [DE]) 24. März 2005 (2005-03-24) * Absatz [0031]; Abbildung 1 * ----- | 1 | |
| X | WO 99/25484 A1 (LOVELACE RESPIRATORY RESEARCH [US]) 27. Mai 1999 (1999-05-27) * Seite 6, Absatz 1 * ----- | 1 | |
| X | US 5 246 163 A (AMANO TOKIMOTO [JP] ET AL) 21. September 1993 (1993-09-21) * Spalte 6, Absatz 1; Abbildung 1 * ----- | 1-3,8,9, 11 | |

RECHERCHIERTE
SACHGEBIETE (IPC)

~~Der vorliegende Recherchenbericht wurde für alle Patentans~~prüche erstellt

| Recherchenort | Abschlußdatum der Recherche | Prüfer |
|---|---|---|
| München | 5. April 2012 | Fischer, Michael |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
.................................................................
& : Mitglied der gleichen Patentfamilie, übereinstimmendes
    Dokument

EPO FORM 1503 03.82 (P04C03)

Europäisches
Patentamt

European
Patent Office

Office européen
des brevets

**Nummer der Anmeldung**

EP 11 19 1787

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

1-3, 8, 9, 11

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches
Patentamt

European
Patent Office

Office européen
des brevets

**MANGELNDE EINHEITLICHKEIT
DER ERFINDUNG
ERGÄNZUNGSBLATT B**

Nummer der Anmeldung

EP 11 19 1787

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-3, 8, 9, 11

   Fluidzerstäuber (1 ) zum Zerstäuben eines Fluids, insbesondere eines Duftstofffluids, umfassend
   - eine Zerstäubungskammer (2) mit mindestens einer Fluidauslassöffnung (211), durch die zerstäubte Fluidtropfen in die Umgebung ausgetragen werden können, und
   - zumindest eine Zweistoff-Düsenanordnung (8), die innerhalb der Zerstäubungskammer (2) untergebracht ist und ein über einen Gaseinlass (3) mit einer Druckgasquelle verbindbares erstes Röhrchen (80) mit einer Gasausströmöffnung (800), durch die ein von der Druckgasquelle zugeführtes, unter Druck stehendes gasförmiges Medium in die Zerstäubungskammer (2) einströmen kann, und ein über einen Fluideinlass (4) mit einem Fluidreservoir verbindbares zweites Röhrchen (81 ) mit einer Fluidaustragöffnung (810), durch die das während des Betriebs des Fluidzerstäubers (1) angesaugte Fluid in die Zerstäubungskammer (2) ausgetragen und durch Wechselwirkung mit dem gasförmigen Medium zerstäubt werden kann, wobei das erste Röhrchen (80) und das zweite Röhrchen (81) geneigt zueinander angeordnet sind, wobei sich die Fluidaustragöffnung (810) über den Rand der Gasausströmöffnung (800) des ersten Röhrchens (80) hinweg erstreckt und eine Längsachse des ersten Röhrchens (80) mit einer Längsachse des zweiten Röhrchens (81 ) einen Winkel > 60[deg.] und < 90[deg.] einschliesst, WOBEI die Gasausströmöffnung (800) des ersten Röhrchens (80) und die Fluidaustragöffnung (810) des zweiten Röhrchens (81) derart zueinander positioniert sind, dass der zur Gasausströmöffnung (800) nächstgelegene Punkt der Fluidaustragöffnung (810) auf einer Projektion des Umfangs der Gasausströmöffnung (800) liegt oder sich bis etwa zum halben Radius innerhalb der Gasausströmöffnung (800) erstreckt.

   ---

2. Anspruch: 4

   Fluidzerstäuber (1 ) zum Zerstäuben eines Fluids, insbesondere eines Duftstofffluids, umfassend
   - eine Zerstäubungskammer (2) mit mindestens einer Fluidauslassöffnung (211), durch die zerstäubte Fluidtropfen in die Umgebung ausgetragen werden können, und
   - zumindest eine Zweistoff-Düsenanordnung (8), die innerhalb der Zerstäubungskammer (2) untergebracht ist und ein über einen Gaseinlass (3) mit einer Druckgasquelle verbindbares erstes Röhrchen (80) mit einer Gasausströmöffnung (800), durch die ein von der Druckgasquelle zugeführtes, unter Druck stehendes gasförmiges Medium in die Zerstäubungskammer (2) einströmen kann, und ein über einen Fluideinlass (4) mit einem Fluidreservoir verbindbares zweites Röhrchen (81 ) mit

20

Europäisches
Patentamt

European
Patent Office

Office européen
des brevets

**MANGELNDE EINHEITLICHKEIT
DER ERFINDUNG
ERGÄNZUNGSBLATT B**

Nummer der Anmeldung

EP 11 19 1787

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

einer Fluidaustragöffnung (810), durch die das während des Betriebs des Fluidzerstäubers (1) angesaugte Fluid in die Zerstäubungskammer (2) ausgetragen und durch Wechselwirkung mit dem gasförmigen Medium zerstäubt werden kann, wobei das erste Röhrchen (80) und das zweite Röhrchen (81) geneigt zueinander angeordnet sind, wobei sich die Fluidaustragöffnung (810) über den Rand der Gasausströmöffnung (800) des ersten Röhrchens (80) hinweg erstreckt und eine Längsachse des ersten Röhrchens (80) mit einer Längsachse des zweiten Röhrchens (81 ) einen Winkel > 60[deg.] und < 90[deg.] einschliesst, WOBEI das erste Röhrchen (80) mindestens einen gebogenen Abschnitt sowie einen sich geradlinig erstreckenden Endabschnitt mit der Gasausströmöffnung (800) umfasst und/oder dass das zweite Röhrchen (81 ) mindestens einen gebogenen Abschnitt sowie einen sich geradlinig erstreckenden Endabschnitt mit der Fluidaustragöffnung (810) umfasst.

---

3. Ansprüche: 5-7

Fluidzerstäuber (1 ) zum Zerstäuben eines Fluids, insbesondere eines Duftstofffluids, umfassend
- eine Zerstäubungskammer (2) mit mindestens einer Fluidauslassöffnung (211), durch die zerstäubte Fluidtropfen in die Umgebung ausgetragen werden können, und
- zumindest eine Zweistoff-Düsenanordnung (8), die innerhalb der Zerstäubungskammer (2) untergebracht ist und ein über einen Gaseinlass (3) mit einer Druckgasquelle verbindbares erstes Röhrchen (80) mit einer Gasausströmöffnung (800), durch die ein von der Druckgasquelle zugeführtes, unter Druck stehendes gasförmiges Medium in die Zerstäubungskammer (2) einströmen kann, und ein über einen Fluideinlass (4) mit einem Fluidreservoir verbindbares zweites Röhrchen (81 ) mit einer Fluidaustragöffnung (810), durch die das während des Betriebs des Fluidzerstäubers (1) angesaugte Fluid in die Zerstäubungskammer (2) ausgetragen und durch Wechselwirkung mit dem gasförmigen Medium zerstäubt werden kann, wobei das erste Röhrchen (80) und das zweite Röhrchen (81) geneigt zueinander angeordnet sind, wobei sich die Fluidaustragöffnung (810) über den Rand der Gasausströmöffnung (800) des ersten Röhrchens (80) hinweg erstreckt und eine Längsachse des ersten Röhrchens (80) mit einer Längsachse des zweiten Röhrchens (81 ) einen Winkel > 60[deg.] und < 90[deg.] einschliesst, WOBEI die Fluidaustragöffnung (810) des zweiten Röhrchens (81) etwa 0,1 bis 0,7 mm, insbesondere 0,2 bis 0,5 mm, vor der Gasausströmöffnung (800) des ersten Röhrchens (80) angeordnet ist.

---

Europäisches
Patentamt

European
Patent Office

Office européen
des brevets

**MANGELNDE EINHEITLICHKEIT
DER ERFINDUNG
ERGÄNZUNGSBLATT B**

**Nummer der Anmeldung**

EP 11 19 1787

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

4. Anspruch: 10

Fluidzerstäuber (1 ) zum Zerstäuben eines Fluids, insbesondere eines Duftstofffluids, umfassend
- eine Zerstäubungskammer (2) mit mindestens einer Fluidauslassöffnung (211), durch die zerstäubte Fluidtropfen in die Umgebung ausgetragen werden können, und
- zumindest eine Zweistoff-Düsenanordnung (8), die innerhalb der Zerstäubungskammer (2) untergebracht ist und ein über einen Gaseinlass (3) mit einer Druckgasquelle verbindbares erstes Röhrchen (80) mit einer Gasausströmöffnung (800), durch die ein von der Druckgasquelle zugeführtes, unter Druck stehendes gasförmiges Medium in die Zerstäubungskammer (2) einströmen kann, und ein über einen Fluideinlass (4) mit einem Fluidreservoir verbindbares zweites Röhrchen (81 ) mit einer Fluidaustragöffnung (810), durch die das während des Betriebs des Fluidzerstäubers (1) angesaugte Fluid in die Zerstäubungskammer (2) ausgetragen und durch Wechselwirkung mit dem gasförmigen Medium zerstäubt werden kann, wobei das erste Röhrchen (80) und das zweite Röhrchen (81) geneigt zueinander angeordnet sind, wobei sich die Fluidaustragöffnung (810) über den Rand der Gasausströmöffnung (800) des ersten Röhrchens (80) hinweg erstreckt und eine Längsachse des ersten Röhrchens (80) mit einer Längsachse des zweiten Röhrchens (81 ) einen Winkel > 60[deg.] und < 90[deg.] einschliesst, WOBEI im Inneren der Zerstäubungskammer (2) oberhalb der Zweistoff-Düsenanordnung (8) ein Trichtermittel (9) angeordnet ist, das zu seinem unteren Ende hin verjüngt ausgebildet ist und an diesem unteren Ende eine Öffnung (90) aufweist.

---

5. Ansprüche: 12-15

Beduftungsvorrichtung (10), umfassend
- ein Gehäuse (11), innerhalb dessen mindestens ein Fluidzerstäuber (1) zum Zerstäuben eines aus einem Duftstofffluidreservoir zugeführten Duftstofffluids, insbesondere eines Duftparfüms oder ätherischen Duftöls, angeordnet ist, und
- mindestens einen Fluidauslass (16), durch den zerstäubte Duftstofffluidtropfen in die Umgebung ausgetragen werden können, WOBEI der mindestens eine Fluidzerstäuber zum Zerstäuben eines Fluids, insbesondere eines Duftstofffluids, umfasst
- eine Zerstäubungskammer (2) mit mindestens einer Fluidauslassöffnung (211), durch die zerstäubte Fluidtropfen in die Umgebung ausgetragen werden können, und
- zumindest eine Zweistoff-Düsenanordnung (8), die innerhalb der Zerstäubungskammer (2) untergebracht ist und ein über einen Gaseinlass (3) mit einer Druckgasquelle verbindbares

Europäisches
Patentamt

European
Patent Office

Office européen
des brevets

**MANGELNDE EINHEITLICHKEIT
DER ERFINDUNG
ERGÄNZUNGSBLATT B**

**Nummer der Anmeldung**

EP 11 19 1787

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

```
erstes Röhrchen (80) mit einer Gasausströmöffnung (800),
durch die ein von der Druckgasquelle zugeführtes, unter
Druck stehendes gasförmiges Medium in die Zerstäubungskammer
(2) einströmen kann, und ein über einen Fluideinlass (4) mit
einem Fluidreservoir verbindbares zweites Röhrchen (81 ) mit
einer Fluidaustragöffnung (810), durch die das während des
Betriebs des Fluidzerstäubers (1) angesaugte Fluid in die
Zerstäubungskammer (2) ausgetragen und durch Wechselwirkung
mit dem gasförmigen Medium zerstäubt werden kann, wobei das
erste Röhrchen (80) und das zweite Röhrchen (81) geneigt
zueinander angeordnet sind, wobei sich die
Fluidaustragöffnung (810) über den Rand der
Gasausströmöffnung (800) des ersten Röhrchens (80) hinweg
erstreckt und eine Längsachse des ersten Röhrchens (80) mit
einer Längsachse des zweiten Röhrchens (81 ) einen Winkel >
60[deg.] und < 90[deg.] einschliesst.
                           ---
```

**ANHANG ZUM EUROPÄISCHEN RECHERCHENBERICHT
ÜBER DIE EUROPÄISCHE PATENTANMELDUNG NR.** EP 11 19 1787

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-04-2012

| Im Recherchenbericht angeführtes Patentdokument | | Datum der Veröffentlichung | Mitglied(er) der Patentfamilie | | | Datum der Veröffentlichung |
|---|---|---|---|---|---|---|
| WO 0248616 | A2 | 20-06-2002 | AU | 3326202 | A | 24-06-2002 |
| | | | CN | 1481260 | A | 10-03-2004 |
| | | | DE | 10062630 | A1 | 29-08-2002 |
| | | | EP | 1215447 | A2 | 19-06-2002 |
| | | | JP | 2004515327 | A | 27-05-2004 |
| | | | US | 2004050948 | A1 | 18-03-2004 |
| | | | WO | 0248616 | A2 | 20-06-2002 |
| DE 3709667 | A1 | 13-10-1988 | KEINE | | | |
| WO 2007073825 | A2 | 05-07-2007 | EP | 1966337 | A2 | 10-09-2008 |
| | | | US | 2007164131 | A1 | 19-07-2007 |
| | | | US | 2011189392 | A1 | 04-08-2011 |
| | | | WO | 2007073825 | A2 | 05-07-2007 |
| WO 2006122561 | A1 | 23-11-2006 | AT | 521414 | T | 15-09-2011 |
| | | | BR | PI0610861 | A2 | 03-08-2010 |
| | | | CN | 101189069 | A | 28-05-2008 |
| | | | DK | 1888249 | T3 | 31-10-2011 |
| | | | EP | 1888249 | A1 | 20-02-2008 |
| | | | ES | 2372412 | T3 | 19-01-2012 |
| | | | JP | 2008540106 | A | 20-11-2008 |
| | | | KR | 20080011220 | A | 31-01-2008 |
| | | | RU | 2375121 | C2 | 10-12-2009 |
| | | | US | 2008311010 | A1 | 18-12-2008 |
| | | | WO | 2006122561 | A1 | 23-11-2006 |
| US 5569073 | A | 29-10-1996 | KEINE | | | |
| EP 0546739 | A2 | 16-06-1993 | AU | 652679 | B2 | 01-09-1994 |
| | | | AU | 2966592 | A | 24-06-1993 |
| | | | CA | 2085213 | A1 | 14-06-1993 |
| | | | DE | 69222125 | D1 | 16-10-1997 |
| | | | DE | 69222125 | T2 | 29-01-1998 |
| | | | EP | 0546739 | A2 | 16-06-1993 |
| | | | ES | 2106834 | T3 | 16-11-1997 |
| | | | JP | 3142671 | B2 | 07-03-2001 |
| | | | JP | 5339582 | A | 21-12-1993 |
| | | | US | 5306418 | A | 26-04-1994 |
| DE 1266697 | B | 18-04-1968 | DE | 1266697 | B | 18-04-1968 |
| | | | FR | 1205646 | A | 03-02-1960 |
| DE 20023948 | U1 | 13-12-2007 | KEINE | | | |
| DE 10339545 | A1 | 24-03-2005 | KEINE | | | |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT
ÜBER DIE EUROPÄISCHE PATENTANMELDUNG NR.**          EP 11 19 1787

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-04-2012

| Im Recherchenbericht angeführtes Patentdokument | Datum der Veröffentlichung | Mitglied(er) der Patentfamilie | Datum der Veröffentlichung |
|---|---|---|---|
| WO 9925484 A1 | 27-05-1999 | AT 549095 T <br> AU 1459299 A <br> DK 1028815 T3 <br> EP 1028815 A1 <br> US 6003512 A <br> WO 9925484 A1 | 15-03-2012 <br> 07-06-1999 <br> 02-07-2012 <br> 23-08-2000 <br> 21-12-1999 <br> 27-05-1999 |
| US 5246163 A | 21-09-1993 | DE 4120409 A1 <br> JP 2066980 C <br> JP 4052377 A <br> JP 7103691 B <br> US 5246163 A | 02-01-1992 <br> 10-07-1996 <br> 20-02-1992 <br> 08-11-1995 <br> 21-09-1993 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 20023948 U1